# EUROPEAN PATENT APPLICATION

(11) **EP 1 114 637 A1**
(43) Date of publication of application: **11.07.2001**
(21) Application number: 00200043.8
(22) Date of filing: 06.01.2000
(51) Int. Cl.: A61K 7/32, A61K 7/16, A61K 7/15, A61K 7/11, A61K 7/06, A61K 7/00

(54) **Aqueous low pressure cosmetic composition comprising a propellant, a thickener and a surfactant**

(71) Applicant: Sara Lee/DE N.V., 3532 AA Utrecht (NL)
(72) Inventor: VOSS, Eckart Karl Heinz, 2566KA Den Haag (NL); KNEBEL, Silke Katharina, 40591 Düsseldorf (DE); MONREAL, Michele, 51515 Kurten (DE); WITHELL, Trevor Keith, 40489 Düsseldorf (DE)
(74) Representative: Ottevangers, Sietse Ulbe

(57) **Abstract**

The invention relates to a cosmetics composition comprising a thickener, a propellant, a surfactant and water, wherein the composition is contained in a container under a pressure of no more than 3 bar.

## Description

The invention relates to a cosmetics composition and to its use.

Cosmetics compositions are nowadays available in many different forms. One product having a very distinct appearance from another product may nevertheless serve the same purpose as said other product. Examples of the wide variety that cosmetics may have include lotions, gels, creams, ointments, milks, aerosols, pastes and so forth.

The present invention seeks to provide a new form for a cosmetics composition. The objective form is a gel which, after application to the skin, creates a post-application foaming effect. When the gel is contacted with for instance skin, it is desired that a noticeable transition takes place from a gel to a dense creamy foam. To achieve this goal, the invention provides a cosmetics composition comprising a thickener, a propellant, a surfactant and water, wherein the composition is contained in a container under a pressure of no more than 3 bar.

Surprisingly, it has been found that a propellant can be incorporated into a cosmetics composition wherein the resultant pressure is less than that of the propellant on its own. Additionally, the propellant is incorporated into the cosmetics composition substantially without affecting the stability of the composition, even if the composition has the form of a gel. When the composition is applied to the skin, the propellant is released from the gel and the user experiences a foam which is very rich, creamy and longlasting. It has further been found that the composition is non-flammable and as such is associated with a reduced risk for consumers with respect to fire and explosion hazards.

It is to be noted that cosmetics compositions in the form of a gel and comprising a propellant are known per se. In the field of shaving creams, gels are marketed which, due to the presence of a propellant, convert into a foaming layer or lather when brought into contact with the skin. More recently, this technology has been modified to allow shower foam products to be marketed. These compositions are generally packaged in containers having two compartments, e.g. a bag made of a laminated material suspended inside an aerosol can. Moreover, the amount of propellant needed in these compositions is relatively high. Hence, shaving gels are packed into pressurized containers wherein the pressure usually is as high as 8 bar or more. Consequently, the container needs to be particularly strong and is therefore made of metal.

A composition according to the invention does not require such high amounts of propellant. As has been mentioned, the pressure in a container in which the composition is contained may be as low as 3 bar or less. Preferably, said pressure is lower than 2 bar. Due to this low pressure, the present composition may advantageously be packed in a plastic container, e.g. a polyethylene or polypropylene container, which is economically much more attractive than package in a metal container. Furthermore, a composition according to the invention has been found to have a lower pressure at elevated temperatures than expected. This feature is advantageous in that it can often not be avoided that the product needs to be stored at elevated temperatures for a certain period of time.

It is preferred that the present composition has the form of a gel. Accordingly, the composition comprises a thickener, which is preferably present in an amount of from 0.01 to 4 wt.%, more preferably from 0.5 to 3 wt.%, based on the weight of the composition. Suitable thickeners are chosen for the compatibility with the propellant and for their capability to provide a stable gel. Examples of preferred thickeners include gums and poly(meth)acrylates, such as polyacyrlic acid. Particularly stable gels have been obtained using xanthan gom as the thickener. The presence of a molecular network is highly beneficial to the stability of the gel and can be demonstrated in rheological measurements.

A further important component of the present composition is a propellant. Advantageously, the propellant is chosen for its physico-chemical properties and the character of the foam texture produced on application to skin. A further consideration on which the choice for a suitable propellant may be based is its environmentally friendly character. Preferred propellants are hydrocarbon gases having from 4 to 7 carbon atoms. Particularly good results have been obtained using isopentane as a propellant. The propellant is preferably present in an amount adjusted to achieve the desired pressure of the composition in a container in which it is packed. Suitable amounts range from 1 to 15 wt.%, more preferably from 4 to 8 wt.%, based on the weight of the composition.

The composition further comprises one or more surfactants. Preferably, the surfactant or surfactants are foaming and skin friendly. Examples of suitable surfactants include polysorbate 20 or 40, coco glucoside, lauryl glucoside, decyl glucoside, lauryl sulfates such as ammonium, sodium, magnesium, MEA, TEA, or Mipa lauryl sulfate, cocamidopropyl betain, and sodium alkyl sulfosuccinates. The surfactant is preferably present in an amount of from 2 to 15 wt.%, preferably from 8 to 13 wt.%, based on the weight of the composition.

Depending on the envisaged purpose of the composition, one or more other ingredients may be present. Examples of such ingredients include preservatives, pH regulating agents, perfumes, anti-bacterial agents, moisturizing agents, UV-filters, and emollients. The composition is formulated to be gel that transforms upon dispensing from a container into a soft and foamy mousse which cleans in a soft and silky manner.

The pH of the composition is preferably regulated to be close to the pH of the skin itself. Accordingly, the pH of the composition is preferably slightly acidic, e.g. in the range of 5 to 8. An example of a suitable pH regulating agents is citric acid. The skilled person will be aware of numerous suitable pH regulating agents that may be employed in the present type of compositions. The amount of the pH regulating agent present is of course adjusted so that the desired pH is achieved.

An emollient or moisturizing agent may be present in order to improve the ease of application of the composition and the final skin feel the user experiences. Examples of suitable emollients or moisturizing agents include glycerin, propenylglycol, PEG 7 glyceryl cocoate, PEG 6 caprylic or capric glycerides, glyceryl oleate and lipids in general, such as paraffin oil or polar oils. An emollient or moisturizing agent is preferably present in an amount ranging from 0.5 to 15 wt.%, based on the weight of the composition.

The balance of the composition will generally be made up by water. Optionally, a small amount of an alcohol, such as ethanol or isopropanol may be present, e.g. to achieve a disinfecting effect. Water will typically be present in an amount ranging from 50 to 95 wt.%, based on the weight of the composition.

Dependent on the chosen ingredients of the composition as set forth above, a cosmetics composition according to the invention may find application as a sun cream or lotion, body milk, shampoo, bathing or shower gel, or moisturizing cream. If desired, the present composition may also be employed in a pharmaceutical setting, for instance as an ointment. In such a case, the composition will further comprise a pharmaceutically active agent or a bioactive agent.

The invention will now be elucidated by the following, non-restrictive examples.

### Example

A composition was prepared of the following ingredients in the following amounts (wt.%):
- Surfactants: Magnesium Laureth Sulfate (1) 11.43
   Lauryl Glucoside (2) 5.19
- pH regulator: Citric acid 0.11
- Preservative: Kathon CG 0.06
- Thickener: Xanthan gom 0.80
- Moisturizing agent: Glycerin 5.00
- Emollient: Cetiol HE 2.00
- Conditioning agent: Merquat Plus 3331 1.00
- Perfume 1.00
- Coloring agent: Patentblue V E 131 0.0015
- Water Balance

The composition was prepared by first adding the water to a vessel. Next, in subsequent order, the preserative and the thickener were added. These components were mixed and homogenized until the thickener was swollen and fully dispersed. To the obtained dispersion, the surfactants were added separately with mixing to fully disperse the surfactant after each addition. The remaining ingredients, except the citric acid, were then added and mixing was continued until all were fully dispersed. Finally, the pH was adjusted by addition of the citric acid.

This composition was then cooled to below 10°C. The propellant to be added, isopentane, was also cooled to said temperature. The composition and the propellant were mixed with one another while taking care that no air was incorporated at constant temperature. The propellant was added in an amount to finally reach a concentration of 6 wt.%, with respect to the total weight of the final composition. After thorough mixing, the composition was allowed to warm up and brought into a suitable plastic container while still having a temperature below 20°C.

## Claims

1. Cosmetics composition comprising a thickener, a propellant, a surfactant and water, wherein the composition is contained in a container under a pressure of no more than 3 bar.

2. Composition according to claim 1, wherein the pressure is no more than 2 bar.

3. Composition according to claim 1 or 2, wherein the container is a plastic container.

4. Composition according to any of the preceding claims, wherein the propellant is a hydrocarbon having from 4 to 7 carbon atoms.

5. Composition according to claim 4, wherein the propellant is isopentane.

6. Composition according to any of the preceding claims, comprising from 0.01 to 4 wt.% of the thickener, from 1 to 15 wt.% of the propellant, from 2 to 15 wt.% of the surfactant and the balance being water.

7. Composition according to any of the preceding claims, wherein the thickener is chosen from the group of gums and poly(meth)acrylate thickeners.

8. Composition according to claim 7, wherein the thickener is xanthan gom.

9. Composition according to any of the preceding claims further comprising one or more ingredients chosen from the group of preservatives, pH regulating agents, perfumes, anti-bacterial agents, moisturizing agents, emollients and UV-filters.

10. Use of a composition according to claim 9 as a sun cream or lotion, body milk, shampoo, bathing or shower gel, ointment, or moisturizing cream.
